# EUROPEAN PATENT APPLICATION

(11) **EP 2 842 483 A1**
(43) Date of publication of application: **04.03.2015**
(21) Application number: 13781783.9
(22) Date of filing: 22.04.2013
(51) Int. Cl.: A61B 5/04, A61B 5/0245

(54) **ELECTROCARDIOGRAPHIC SIGNAL MEASUREMENT DEVICE AND ELECTROCARDIOGRAPHIC SIGNAL MEASUREMENT METHOD**

(30) Priority: 25.04.2012 JP 2012099784
(71) Applicant: Murata Manufacturing Co., Ltd., Nagaokakyo-shi, Kyoto 617-8555 (JP)
(72) Inventor: SHIMUTA, Toru, Nagaokakyo-shi Kyoto 617-8555 (JP)
(74) Representative: Reeve, Nicholas Edward
(86) International application number: PCT/JP2013/061720
(87) International publication number: WO 2013/161729

(57) **Abstract**

Provided is an electrocardiographic signal measurement apparatus that is easily attached or detached and is capable of stably measuring electrocardiographic signals all the time without obstructing motions of a user.

An electrocardiographic signal measurement apparatus (1) includes; an outer ear electrode (11) that is made of a material having elasticity and conductivity and is brought into contact with and attached to an area of an outer ear including a boundary region between an auricle and an earlobe configuring the outer ear, an upper arm electrode (15) that is brought into contact with and attached to an upper arm on the opposite side in the horizontal direction to the side where the outer ear electrode (11) is attached to the outer ear, and a signal processing unit (51) that is connected with the outer ear electrode (11) and the upper arm electrode (15) and measures an electrocardiographic signal detected by the outer ear electrode (11) and the upper arm electrode (15).

## Description

### Technical Field

The present invention relates to electrocardiographic signal measurement apparatuses and electrocardiographic signal measurement methods.

### Background Art

In recent years, people have been paying more attention to maintenance and improvement of their health than before. As such, people want to readily obtain health-related information such as an electrocardiogram, a heart rate, and so on for health care in daily life. Here, Patent Document 1 discloses a heart rate measurement apparatus configured to measure a heart rate from a chest portion. This heart rate measurement apparatus includes; a pair of electrodes that is secured to flat and flexible fixed plates made of rubber or the like and collects an electrocardiographic signal, and a belt for fixing the pair of electrodes. In measuring the electrocardiographic signal, the pair of electrodes is brought into close contact with the skin of the chest portion and the belt is wound thereupon to fix the electrodes.

Patent Document 2 discloses a bio-information measurement shirt. This bio-information measurement shirt, which is intended to be worn on the upper body of a test subject, includes electrodes at positions in the shirt corresponding to the chest portion and the limb portions, and sensors at positions in the shirt corresponding to the waist portion and the abdomen portion. To be more specific, the limb electrodes are disposed, when the shirt is worn, at the positions to cover a body surface near the collarbone of the test subject as well as a body surface near the pelvis thereof. The chest electrodes are disposed, when the shirt is worn, at the positions to cover a body surface ranging from the vicinity of the front of the sternum of the test subject to the vicinity of a side portion of the left chest thereof in a body axis vertical direction and ranging from the vicinity of the fourth rib to the vicinity of the sixth rib in the body axis direction.

Patent Document 3 discloses a mobile bio-data measurement apparatus. This mobile bio-data measurement apparatus includes a positive electrode, a GND electrode, and a negative electrode on side surfaces of a housing formed in a parallelepiped shape. According to this mobile bio-data measurement apparatus, by applying the positive electrode to the chest portion while holding the apparatus such that four fingers other than a thumb (from an index finger to a little finger) of the right hand are placed on the GND electrode and the thumb of the right hand is placed on the negative electrode, a voltage value between the positive electrode and the GND electrode as well as a voltage value between the GND electrode and the negative electrode can be measured so that an electrocardiographic wave and a heart rate can be measured.

Patent Document 4 discloses an electronic wristwatch-type signal detection apparatus. This signal detection apparatus has an electrocardiographic wave (ECG wave) detection electrode on the front surface of a wristwatch housing. Meanwhile, the rear surface of the wristwatch housing is covered with the other electrocardiographic wave detection electrode that constitutes a pair of the electrodes together with the above electrocardiographic wave detection electrode. An electrocardiographic wave detector is electrically connected with both the electrocardiographic wave detection electrodes, and detects the electrocardiographic wave (ECG wave) from a fingertip of the right hand which is put on the electrocardiographic wave detection electrode and the left wrist which is in contact with the other electrocardiographic wave detection electrode.

Patent Document 5 discloses a bio-information measurement sensor capable of measuring bio-information with certainty even when massage treatment being performed. The bio-information measurement sensor provided in a massage machine includes; a headphone provided with an earlobe sensor having a heart rate measurement electrode and attached to one of the earlobes, and a finger-attachment probe having a heart rate measurement electrode and attached to a finger.

Patent Document 6 discloses a bio-information detection apparatus configured to collect an electrocardiographic waveform from a potential of an ear canal and a potential of a palm. This bio-information detection apparatus includes a first electrode formed on a probe, a second electrode configured on a surface of a main body of the apparatus with which the palm makes contact, and an electrocardiographic signal processing means in which an electrocardiographic waveform is detected and processed based on difference in potential between these two electrodes. According to this bio-information detection apparatus, a potential of a skin surface at the inside of an ear canal or in the vicinity of an entrance to the ear canal is detected when the probe is inserted into the ear canal; meanwhile, part of the palm holding the main body makes direct contact with the electrode on the main body so that a potential of the palm is detected. A potential difference between the two portions of the test subject body obtained in the manner described above is amplified and outputted so as to collect the electrocardiographic waveform.

Patent Document 7 discloses an earphone-type heart rate alarm apparatus. This heart rate alarm apparatus is configured of electrodes that are attached to both ears of a test subject and cause to derive an electromotive force with electrophysiology of the heart, and of a heart rate detector electrically connected with the above electrodes.

### Citation List

### Patent Document

Patent Document 1: Japanese Unexamined Patent Application Publication No. 60-92734
Patent Document 2: Japanese Unexamined Patent Application Publication No. 2011-167546
Patent Document 3: Japanese Unexamined Patent Application Publication No. 2003-144403
Patent Document 4: Japanese Unexamined Patent Application Publication No. 2000-316824
Patent Document 5: Japanese Unexamined Patent Application Publication No. 2002-576
Patent Document 6: Japanese Unexamined Patent Application Publication No. 2000-217792
Patent Document 7: Japanese Unexamined Patent Application Publication No. 61-279222

### Summary of Invention

### Technical Problem

In the case where an electrocardiographic signal is intended to be measured using the heart rate measurement apparatus disclosed in Patent Document 1 or the bio-information measurement shirt disclosed in Patent Document 2, clothes of the test subject need be taken off when attaching or detaching the electrocardiographic electrodes. Accordingly, it takes a long time to attach or detach the electrocardiographic electrodes.

In the cases of the mobile bio-data measurement apparatus disclosed in Patent Document 3, the electronic wristwatch-type signal detection apparatus disclosed in Patent Document 4, the bio-information measurement sensor disclosed in Patent Document 5, and the bio-information detection apparatus disclosed in Patent Document 6, at least one of the electrodes need be in contact with a finger or a palm, which causes a test subject to take a fixed posture and consequently makes it difficult to carry out the measurement while the test subject making some other motions (for example, taking exercise or the like).

In the case of the earphone-type heart rate alarm apparatus disclosed in Patent Document 7, an electrocardiographic signal is measured with the electrodes attached to both ears. However, in the case where the measurement is carried out with both the ears, because an electrocardiographic signal becomes extremely weak, the measurement is significantly affected by radiation noise from ambient environment, body motion noise, and so on. This makes it difficult to stably measure the electrocardiographic signal.

The present invention has been made to solve the above-mentioned problems, and it is an object of the present invention to provide an electrocardiographic signal measurement apparatus and an electrocardiographic signal measurement method capable of being attached/detached with ease and also capable of measuring an electrocardiographic signal in a stable manner all the time without obstructing motions of a user.

### Solution to Problem

An electrocardiographic signal measurement apparatus according to an aspect of the present invention includes an outer ear electrode that is in contact with and attached to an outer ear, an upper arm / shoulder electrode that is in contact with and attached to an upper arm or a shoulder, and a measurement means that is connected with the outer ear electrode and the upper arm / shoulder electrode and measures an electrocardiographic signal detected by the outer ear electrode and the upper arm / shoulder electrode.

An electrocardiographic signal measurement method according to an aspect of the present invention includes an outer ear electrode attaching step in which an outer ear electrode is brought into contact with and attached to an outer ear, an upper arm / shoulder electrode attaching step in which an upper arm / shoulder electrode is brought into contact with and attached to an upper arm or a shoulder, and a measuring step in which an electrocardiographic signal detected by the outer ear electrode and the upper arm / shoulder electrode is measured.

According to the electrocardiographic signal measurement apparatus and the electrocardiographic signal measurement method of the aspects of the present invention, because electrocardiographic electrodes (the outer ear electrode and the upper arm / shoulder electrode) are attached to an outer ear and an upper arm or a shoulder, clothes of a user need not be taken off when attaching/detaching the electrocardiographic electrodes, whereby the attaching/detaching will not take a long time. In addition, because the electrocardiographic electrodes are attached to an outer ear and an upper arm or a shoulder, both hands are free, which makes it possible to carry out the measurement while the user making some other motions (for example, taking exercise or the like). Moreover, because the electrocardiographic electrodes are attached to an outer ear and an upper arm or a shoulder, an electrocardiographic signal that is detected is relatively large and resistant to radiation noise from ambient environment, body motion noise, and so on, thereby making it possible to stably carry out the measurement. As a result, electrocardiographic signals can be measured always in a stable manner without obstructing motions of the user, and attaching/detaching the electrodes can be made with ease.

In the electrocardiographic signal measurement apparatus according to the aspect of the present invention, it is preferable for the outer ear electrode to be in contact with and attached to an area of an outer ear including a boundary region between an auricle and an earlobe configuring the outer ear.

In the electrocardiographic signal measurement method according to the aspect of the present invention, it is preferable for the outer ear electrode to be brought into contact with and attached to an area of the outer ear including a boundary region between an auricle and an earlobe configuring the outer ear in the outer ear electrode attaching step.

In this case, stability of the measurement can be improved because the outer ear electrode is attached to an outer ear, in particular, to a portion in the vicinity of a boundary between the earlobe and the auricle. In other words, variations in detected signals depending on individuals are smaller in comparison with a case in which the outer electrode is attached to an earlobe whose shape considerably varies depending on individuals. Further, because the outer ear electrode is attached to an area near a relatively hard auricle, the state of contact of the outer ear electrode is stable even when the test subject taking exercise in comparison with a case in which the outer ear electrode is attached to a softer earlobe. Accordingly, the stability of measurement of electrocardiographic signals can be improved.

Especially, it is preferable for the outer ear electrode to be made of a material having elasticity and conductivity and formed so as to bend along a contour of the outer ear. By doing so, because the outer ear electrode can be brought into close contact with the outer ear, noise generated due to body motion or the like can be reduced so as to more stably obtain electrocardiographic signals.

In the electrocardiographic signal measurement apparatus according to the aspects of the present invention, it is preferable for the upper arm / shoulder electrode to be in contact with and attached to an upper arm or a shoulder on the opposite side in the horizontal direction to the side where the outer ear electrode is attached to the outer ear.

In the electrocardiographic signal measurement method according to the aspects of the present invention, it is preferable for the upper arm / shoulder electrode to be brought into contact with and attached to an upper arm or a shoulder on the opposite side in the horizontal direction to the side where the outer ear electrode is attached to the outer ear in the upper arm / shoulder electrode attaching step.

In general, in the case where the outer ear electrode and the upper arm / shoulder electrode are attached at different sides opposing each other in the horizontal direction, amplitude of an electrocardiographic signal is normally larger compared to a case in which both the electrocardiographic electrodes are attached at the left side of the test subject body (the left outer ear and the left upper arm or shoulder) or the right side thereof (the right outer ear and the right upper arm or shoulder). In this case, because the upper arm / shoulder electrode is in contact with and attached to an upper arm or a shoulder on the opposite side in the horizontal direction to the side of the outer ear to which the outer ear electrode is attached, amplitude of a detected electrocardiographic signal is larger so that the electrocardiographic signal measurement can be more stably carried out.

In particular, it is preferable for the upper arm / shoulder electrode to be made of a material having elasticity and conductivity and formed so as to be in close contact with and attached to an upper arm or a shoulder. By doing so, because the upper arm / shoulder electrode can be brought into close contact with the upper arm or the shoulder, noise generated due to body motion or the like can be reduced so as to more stably obtain electrocardiographic signals.

It is preferable for the electrocardiographic signal measurement apparatus according to the aspects of the present invention to further include a photoelectric pulse wave sensor that is provided in the vicinity of the outer ear electrode or the upper arm / shoulder electrode and detects a photoelectric pulse wave signal, and for the measurement means to measure an electrocardiographic signal and complement the electrocardiographic signal using the photoelectric pulse wave signal detected by the photoelectric pulse wave sensor.

In this case, by the combining and using the photoelectric pulse wave sensor, even if an electrocardiographic signal is hard to be measured due to, for example, an individual physical constitution, a state of contact between the skin and the electrocardiographic electrode, and/or influence of disturbance noise, and so on, it is possible to complement a measurement result of electrocardiographic signal using a measurement result obtained by the photoelectric pulse wave sensor.

An electrocardiographic signal measurement apparatus according to an aspect of the present invention includes a first outer ear electrode and a second outer ear electrode that are electrically connected with each other and are in contact with and attached to left and right outer ears, an upper arm / shoulder electrode that is in contact with and attached to an upper arm or a shoulder, and a measurement means that is connected with the first outer ear electrode, the second outer ear electrode, and the upper arm / shoulder electrode so as to measure an electrocardiographic signal detected by the first outer ear electrode, the second outer ear electrode, and the upper arm / shoulder electrode.

An electrocardiographic signal measurement method according to an aspect of the present invention includes; an outer ear electrode attaching step in which a first outer ear electrode and a second outer ear electrode electrically connected with each other are respectively brought into contact with and attached to left and right outer ears, an upper arm / shoulder electrode attaching step in which an upper arm / shoulder electrode is brought into contact with and attached to an upper arm or a shoulder, and a measuring step in which measured is an electrocardiographic signal detected by the first outer ear electrode, the second outer ear electrode, and the upper arm / shoulder electrode.

In general, as described above, in the case where an outer ear and an upper arm / shoulder to which the electrocardiographic electrodes (the outer ear electrode and the upper arm / shoulder electrode) are respectively attached are positioned on the different sides opposing each other in the horizontal direction, amplitude of an electrocardiographic signal is normally larger compared to a case in which both the electrocardiographic electrodes are attached at the left side of the test subject body or the right side thereof. However, even if the electrocardiographic electrodes are respectively attached at the different sides opposing each other as described above and the measurement is then carried out, there is a case in which a S/N (signal to noise) ratio of the electrocardiographic signal is not good depending on, for example, an individual physical constitution, a state of attachment of the electrocardiographic electrodes, a state of disturbance noise, and so on. Here, in the electrocardiographic signal measurement apparatus and the electrocardiographic signal measurement method according to the aspects of the present invention, two outer ear electrodes (the first and second outer ear electrodes) electrically connected with each other to be at the same potential are respectively brought into contact with and attached to the left and right outer ears, and an electrocardiographic signal is detected between the two outer ear electrodes and the upper arm / shoulder electrode. Because of this, electrocardiographic signals can be measured more stably.

In the electrocardiographic signal measurement method according to the aspects of the present invention, it is preferable for a photoelectric pulse wave sensor for detecting a photoelectric pulse wave signal to be further brought into contact with and attached to an outer ear in the outer ear attaching step, and for an electrocardiographic signal to be measured and complemented using a photoelectric pulse wave signal detected by the photoelectric pulse wave sensor in the measuring step.

In this case, by combining and using the photoelectric pulse wave sensor, even if an electrocardiographic signal is hard to be measured due to, for example, an individual physical constitution, a state of contact between the skin and the electrocardiographic electrodes, and/or influence of disturbance noise, and so on, it is possible to complement a measurement result of electrocardiographic signal using a measurement result obtained by the photoelectric pulse wave sensor.

An electrocardiographic signal measurement apparatus according to an aspect of the present invention includes; a first outer ear electrode that is in contact with and attached to one of the left and right outer ears, a second outer ear electrode that is in contact with and attached to the other outer ear, an upper arm / shoulder electrode that is in contact with and attached to an upper arm or a shoulder, and a measurement means that is connected with the first outer ear electrode, the second outer ear electrode, and the upper arm / shoulder electrode and performs electrocardiographic signal measurement in the manner as follows: that is, of a combination of the first outer ear electrode and the upper arm / shoulder electrode and a combination of the second outer ear electrode and the upper arm / shoulder electrode, the combination that detects an electrocardiographic signal with a larger SN ratio is selected and the electrocardiographic signal is then measured.

An electrocardiographic signal measurement method according to an aspect of the present invention includes; a first outer ear electrode attaching step in which a first outer ear electrode is brought into contact with and attached to one of the left and right outer ears, a second outer ear electrode attaching step in which a second outer ear electrode is brought into contact with and attached to the other outer ear, an upper arm / shoulder electrode attaching step in which an upper arm / shoulder electrode is brought into contact with and attached to an upper arm or a shoulder, and a measuring step in which electrocardiographic signal measurement is performed in the manner as follows: that is, of a combination of the first outer ear electrode and the upper arm / shoulder electrode and a combination of the second outer ear electrode and the upper arm / shoulder electrode, the combination that detects an electrocardiographic signal with a larger SN ratio is selected and the electrocardiographic signal is then measured.

As described above, even if the electrocardiographic electrodes are respectively attached at the different sides opposing each other in the horizontal direction and the measurement is then carried out, there is a case in which a S/N ratio of the electrocardiographic signal is not good depending on, for example, an individual physical constitution, a state of attachment of the electrocardiographic electrodes (the outer ear electrode and the upper arm / shoulder electrode), a state of disturbance noise, and so on. Here, in the electrocardiographic signal measurement apparatus and the electrocardiographic signal measurement method according to the aspects of the present invention, a SN ratio of the electrocardiographic signal between the right outer ear and an upper arm or a shoulder and a SN ratio of the electrocardiographic signal between the left outer ear and the upper arm or the shoulder are compared with each other, and of the two combinations, the combination that exhibits a larger SN ratio is selected and the electrocardiographic signal is then measured. Because of this, electrocardiographic signals can be more stably measured.

In this case, it is preferable for the electrocardiographic signal measurement apparatus according to the aspects of the present invention to further include a photoelectric pulse wave sensor that is provided in the vicinity of the first outer ear electrode, the second outer ear electrode, or the upper arm / shoulder electrode and detects a photoelectric pulse wave signal, and for the measurement means to measure an electrocardiographic signal and complement the electrocardiographic signal using a photoelectric pulse wave signal detected by the photoelectric pulse wave sensor.

An electrocardiographic signal measurement apparatus according to an aspect of the present invention includes a forehead electrode that is in contact with and attached to a forehead, an upper arm / shoulder electrode that is in contact with and attached to an upper arm or a shoulder, and a measurement means configured to measure an electrocardiographic signal detected by the forehead electrode and the upper arm / shoulder electrode.

An electrocardiographic signal measurement method according to an aspect of the present invention includes a forehead electrode attaching step in which a forehead electrode is brought into contact with and attached to a forehead, an upper arm / shoulder electrode attaching step in which an upper arm / shoulder electrode is brought into contact with and attached to an upper arm or a shoulder, and a measuring step in which an electrocardiographic signal detected by the forehead electrode and the upper arm / shoulder electrode is measured.

In the electrocardiographic signal measurement apparatus and the electrocardiographic measurement method according to the aspects of the present invention, because the electrocardiographic electrodes (the forehead electrode and the upper arm / shoulder electrode) are respectively attached to the forehead and an upper arm or a shoulder, it is unnecessary for a user to take off the clothes when attaching/detaching the electrocardiographic electrodes, whereby the attaching/detaching will not take a long time. In addition, because the electrocardiographic electrodes are attached to the forehead and an upper arm or a shoulder, both hands are free, which makes it possible to carry out the measurement while making some other motions (for example, taking exercise or the like). Moreover, because the electrocardiographic electrodes are attached to the forehead and an upper arm or a shoulder, an electrocardiographic signal that is detected is relatively large and resistant to radiation noise from ambient environment, body motion noise, and so on, thereby making it possible to stably carry out the measurement. As a result, electrocardiographic signals can be measured in a stable manner all the time without obstructing motions of a user, and attaching/detaching the electrodes can be made with ease.

In this case, it is preferable for the electrocardiographic signal measurement apparatus according to the aspect of the present invention to further include a photoelectric pulse wave sensor that is provided in the vicinity of the forehead electrode or the upper arm / shoulder electrode and detects a photoelectric pulse wave signal, and for the measurement means to measure an electrocardiographic signal and complement the electrocardiographic signal using a photoelectric pulse wave signal detected by the photoelectric pulse wave sensor.

An electrocardiographic signal measurement apparatus according to an aspect of the present invention includes a first upper arm / shoulder electrode that is in contact with and attached to one of the left and right upper arms or shoulders, a second upper arm / shoulder electrode that is in contact with and attached to the other upper arm or shoulder, and a measurement means configured to measure an electrocardiographic signal detected by the first upper arm / shoulder electrode and the second upper arm / shoulder electrode.

An electrocardiographic signal measurement method according to an aspect of the present invention includes a first upper arm / shoulder electrode attaching step in which a first upper arm / shoulder electrode is brought into contact with and attached to one of the left and right upper arms or shoulders, a second upper arm / shoulder electrode attaching step in which a second upper arm / shoulder electrode is brought into contact with and attached to the other upper arm or shoulder, and a measuring step in which an electrocardiographic signal detected by the first upper arm / shoulder electrode and the second upper arm / shoulder electrode is measured.

In the electrocardiographic signal measurement apparatus and the electrocardiographic measurement method according to the aspects of the present invention, because the electrocardiographic electrodes (the first upper arm / shoulder electrode and the second upper arm / shoulder electrode) are attached to the left and right upper arms or shoulders, it is unnecessary for a user to take off the clothes when attaching/detaching the electrocardiographic electrodes, whereby the attaching/detaching will not take a long time. In addition, because the electrocardiographic electrodes are attached to the left and right upper arms or shoulders, both hands are free, which makes it possible to carry out the measurement while making some other motions (for example, taking exercise or the like). Moreover, because the electrocardiographic electrodes are attached to the left and right upper arms or shoulders, an electrocardiographic signal that is detected is relatively large and resistant to radiation noise from ambient environment, body motion noise, and so on, thereby making it possible to stably carry out the measurement. As a result, electrocardiographic signals can be measured in a stable manner all the time without obstructing motions of a user, and attaching/detaching the electrodes can be made with ease.

In this case, it is preferable for the electrocardiographic signal measurement apparatus according to the aspect of the present invention to further include a photoelectric pulse wave sensor that is provided in the vicinity of the first upper arm / shoulder electrode or the second upper arm / shoulder electrode and detects a photoelectric pulse wave signal, and for the measurement means to measure an electrocardiographic signal and complement the electrocardiographic signal using a photoelectric pulse wave signal detected by the photoelectric pulse wave sensor.

### Advantageous Effects of Invention

According to the aspects of the present invention, electrocardiographic signals can be measured in a stable manner all the time without obstructing motions of a user, and attaching/detaching the electrodes can be made with ease.

### Brief Description of Drawings

[Fig. 1] Fig. 1 is a block diagram illustrating a configuration of an electrocardiographic signal measurement apparatus according to a first embodiment of the present invention.
[Fig. 2] Fig. 2 is a diagram illustrating a mounting example of the electrocardiographic signal measurement apparatus according to the first embodiment.
[Fig. 3] Fig. 3 is a diagram illustrating a configuration of an outer ear clip having an outer ear electrode and a photoelectric pulse wave sensor.
[Fig. 4] Fig. 4 is another mounting example of the electrocardiographic signal measurement apparatus according to the first embodiment.
[Fig. 5] Fig. 5 is a block diagram illustrating a configuration of an electrocardiographic signal measurement apparatus according to a second embodiment.
[Fig. 6] Fig. 6 is a block diagram illustrating a configuration of an electrocardiographic signal measurement apparatus according to a third embodiment.
[Fig. 7] Fig. 7 is a block diagram illustrating a configuration of an electrocardiographic signal measurement apparatus according to a fourth embodiment.
[Fig. 8] Fig. 8 is a diagram illustrating a mounting example of the electrocardiographic signal measurement apparatus according to the fourth embodiment.
[Fig. 9] Fig. 9 is a block diagram illustrating a configuration of an electrocardiographic signal measurement apparatus according to a fifth embodiment.
[Fig. 10] Fig. 10 is a diagram illustrating a mounting example of the electrocardiographic signal measurement apparatus according to the fifth embodiment.
[Fig. 11] Fig. 11 is a diagram illustrating a mounting example of an electrocardiographic signal measurement apparatus according to a variation.

### Description of Embodiments

Hereinafter, preferred embodiments of the present invention will be described in detail with reference to the drawings. Note that in the drawings, same constituent elements are given the same reference signs and duplicate description thereof will be omitted.

### [First Embodiment]

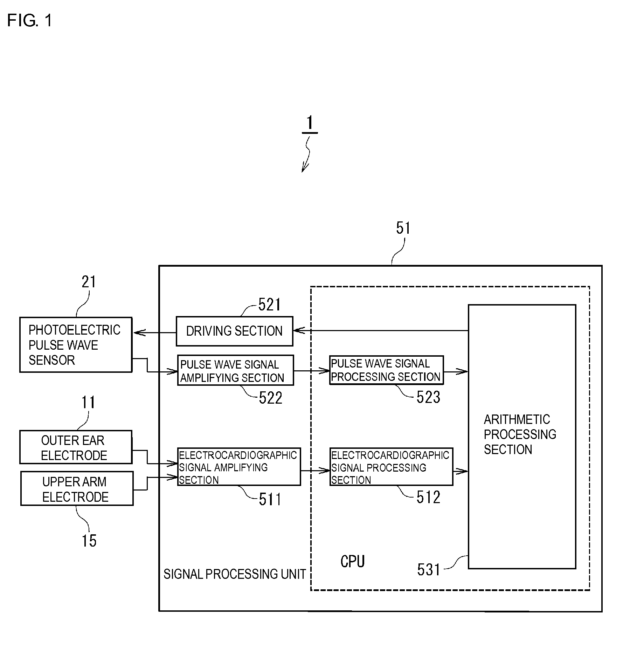

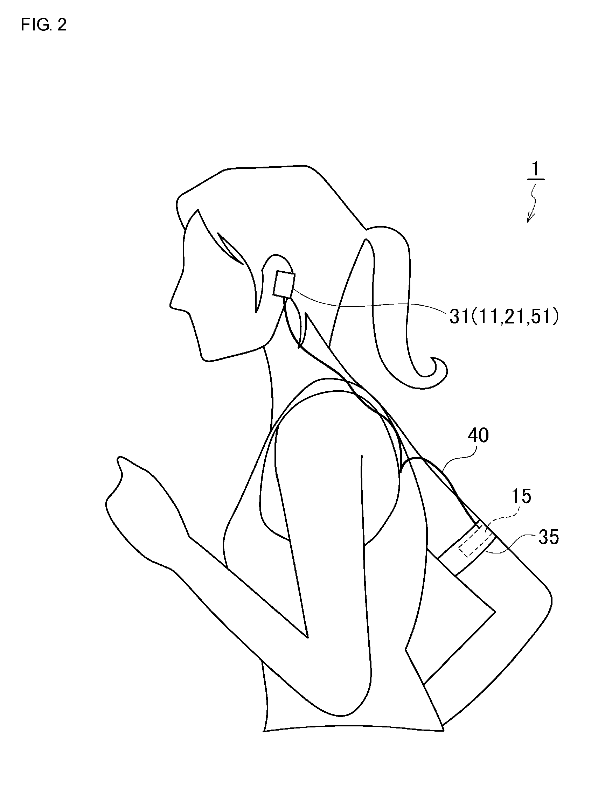

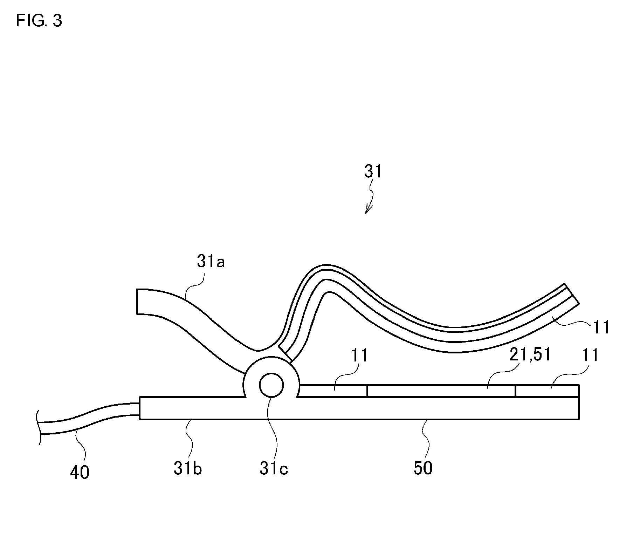

First, referring to Figs. 1 through 3, a configuration of an electrocardiographic signal measurement apparatus 1 according to a first embodiment of the present invention will be described. Fig. 1 is a block diagram illustrating the configuration of the electrocardiographic signal measurement apparatus 1. Fig. 2 is a diagram illustrating a mounting example of the electrocardiographic signal measurement apparatus 1. Fig. 3 is a diagram illustrating a configuration of an outer ear clip 31 having an outer ear electrode 11 and a photoelectric pulse wave sensor 21.

The electrocardiographic signal measurement apparatus 1 is configured so that an electrocardiographic signal and a photoelectric pulse wave signal can be measured while a user making daily-life motions or doing daily-life activities (for example, taking exercise or the like). The electrocardiographic signal measurement apparatus 1 includes the outer ear electrode 11 and an upper arm electrode 15 (corresponds to an upper arm / shoulder electrode disclosed in the appended claims) which are electrocardiographic electrodes configured to detect an electrocardiographic signal, the photoelectric pulse wave sensor 21 configured to detect a photoelectric pulse wave signal, and a signal processing unit 51 configured to measure a heart rate, a pulse rate, and so on based on a detected electrocardiographic signal and a detected photoelectric pulse wave signal. Hereinafter, each constituent element thereof will be described in detail.

The outer ear electrode 11 is brought into contact with and attached to one of the left and right outer ears of a user. It is preferable for the outer ear electrode 11 to be in contact with and attached to an area of the outer ear including a boundary region between an auricle and an earlobe configuring the outer ear. Although the outer ear electrode 11 is attached to the left ear in Fig. 2, it may be attached to the right ear. In order for the outer ear electrode 11 to be in close contact with and attached to an outer ear (a portion near the boundary between the auricle and the earlobe), the outer ear electrode 11 is made of a material having elasticity and conductivity and formed in a wave shape so as to bend along a contour of the outer ear (see Fig. 3). Accordingly, as an electrode material of the outer ear electrode 11, conductive gel, conductive rubber, conductive cloth, or the like is preferably used, for example. Further, for example, conductive plastic, metal (those having high corrosion-resistance and unlikely to cause metal allergy are desirable, such as stainless steel, Au, titanium, and so on), a capacitive coupling electrode, or the like can be also used as the electrode material of the outer ear electrode 11.

The outer ear electrode 11 is, as shown in Fig. 3, provided on the inner surface side of the outer ear clip 31 that is formed to be freely openable/closable via a hinge 31c, so that the outer ear electrode 11 can be brought into close contact with and attached to an outer ear (a portion near the boundary between the auricle and the earlobe). Accordingly, by pinching an area of the outer ear ranging from the auricle to the earlobe with the outer ear clip 31, the outer ear electrode 11 can be brought into close contact with and attached to the outer ear.

To be more specific, the outer ear clip 31 has a pair of support members 31a and 31b that is so provided as to be freely openable/closable via the hinge 31c, that is, has the support member 31a that is formed in a wave shape so as to bend along the shape extending from the auricle to the earlobe and the support member 31b substantially formed in a plate-like shape. On the inner surface of the support member 31a, the wave-shaped outer ear electrode 11 is provided, as described above, along the shape of the support member 31a. Meanwhile, the support member 31b is configured, in the present embodiment, using a circuit board 50 on which the photoelectric pulse wave sensor 21 is mounted.

Further, on the inner surface of the support member 31b (upper surface of the circuit board 50), the outer ear electrode 11, in addition to the photoelectric pulse wave sensor 21, is provided at a side of (or at a circumferential edge of) the photoelectric pulse wave sensor 21. Note that the outer ear electrode 11 provided on the support member 31a and the outer ear electrode 11 provided on the support member 31b are electrically connected with each other. A spring (not illustrated) is provided to the hinge 31c of the outer ear clip 31, and the spring biases base end portions of the outer ear clip 31 in a direction in which the base end portions thereof are distanced from each other so as to pinch the outer ear with a predetermined pressure.

The photoelectric pulse wave sensor 21 is configured such that a light-emitting device such as an LED, a VCSEL, or the like and a light-receiving device such as a photodiode (PD) or the like are included therein. The photoelectric pulse wave sensor 21 optically detects a photoelectric pulse wave signal making use of absorption characteristics of hemoglobin in the blood when the outer ear clip 31 is attached to an outer ear of the user. In the present embodiment, the signal processing unit 51 and the photoelectric pulse wave sensor 21 are integrally formed as one entity. The outer ear electrode 11 and the photoelectric pulse wave sensor 21 are connected with the signal processing unit 51, and respectively output a detected potential of the outer ear (electrocardiographic signal) and a detected photoelectric pulse wave signal to the signal processing unit 51. The photoelectric pulse wave sensor 21 may be provided in the vicinity of the upper arm electrode 15 (for example, integrated as one entity). Details of the upper arm electrode 15 will be explained below.

The upper arm electrode 15 is so attached as to be in contact with an upper arm of the user. It is preferable for the upper arm electrode 15 to be attached to an upper arm (right upper arm in the example shown in Fig. 2) on the opposite side in the horizontal direction to a side where the outer ear electrode 11 is attached to an ear (left ear in the example shown in Fig. 2). More specifically, the upper arm electrode 15 is made of, for example, conductive rubber, conductive cloth, or the like, and is provided on the inner side of an upper arm belt 35. Accordingly, the upper arm electrode 15 is attached to an upper arm of the user so as to be in contact with the upper arm by winding and attaching the upper arm belt 35 around the upper arm of the user using a hook-and-loop fastener or the like, for example. Note that the upper arm electrode 15 may be pasted on an upper arm of the user. The upper arm electrode 15 is connected with the signal processing unit 51 via a cable 40, and outputs a potential of the upper arm (electrocardiographic signal) to the signal processing unit 51 via the cable 40.

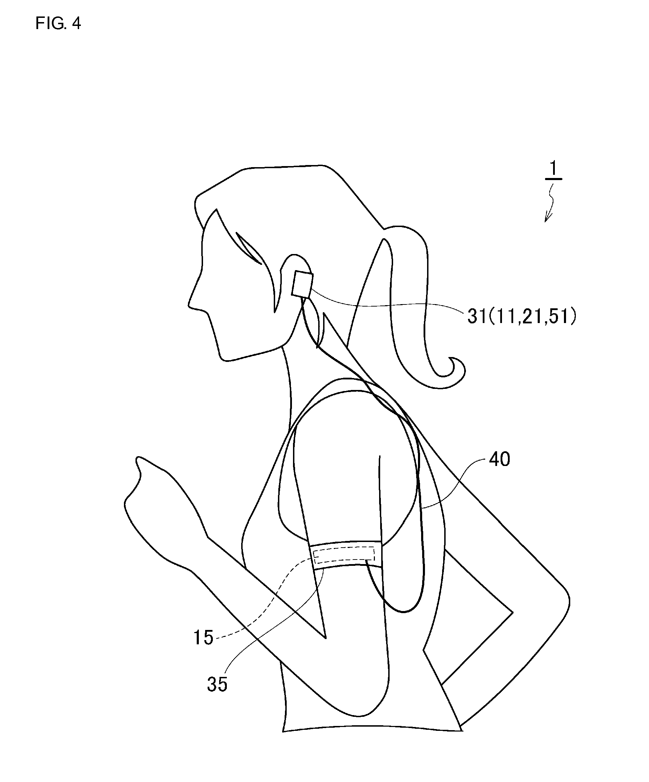

Although Fig. 2 illustrates the case in which the electrocardiographic electrodes (the outer ear electrode 11 and the upper arm electrode 15) are respectively attached to the left ear and the right upper arm, the electrocardiographic electrodes 11 and 15 may be attached to the right ear and the left upper arm, respectively. Here, in general, in the case where the outer ear electrode 11 and the upper arm electrode 15 are attached at different sides opposing each other in the horizontal direction, amplitude of an electrocardiographic signal becomes larger. However, detection conditions differ depending on, for example, an individual physical constitution, a state of attachment of the electrocardiographic electrodes 11 and 15, a state of disturbance noise, and so on. As such, both the electrocardiographic electrodes 11 and 15 may be attached at the same side of the user's body in accordance with the detection conditions. In other words, the electrocardiographic electrodes 11 and 15 may be attached to the left ear and the left upper arm (see Fig. 4), or the right ear and the right upper arm, respectively. Note that Fig. 4 is another mounting example of the electrocardiographic signal measurement apparatus 1.

As described above, the outer ear electrode 11, the upper arm electrode 15, and the photoelectric pulse wave sensor 21 are connected with the signal processing unit 51, and a potential of the outer ear (electrocardiographic signal), a potential of the upper arm (electrocardiographic signal), and a photoelectric pulse wave signal that have been detected are inputted to the signal processing unit 51.

The signal processing unit 51 processes the outer ear potential (electrocardiographic signal) and the upper arm potential (electrocardiographic signal) that have been inputted so as to measure a heart rate and the like. Further, the signal processing unit 51 processes the photoelectric pulse wave signal having been inputted so as to measure a pulse rate and the like, and complements the electrocardiographic signal using a result of the processing. In other words, the signal processing unit 51 functions as a measurement means disclosed in the appended claims. As such, the signal processing unit 51 includes an electrocardiographic signal amplifying section 511, an electrocardiographic signal processing section 512, a driving section 521, a pulse wave signal amplifying section 522, a pulse wave signal processing section 523, and an arithmetic processing section 531.

The electrocardiographic signal amplifying section 511 is configured with a differential amplifier using an operational amplifier or the like, for example, and amplifies a potential difference (electrocardiographic signal) between the outer ear electrode 11 and the upper arm electrode 15. The electrocardiographic signal amplified by the electrocardiographic signal amplifying section 511 undergoes A/D conversion, thereafter is outputted to the electrocardiographic signal processing section 512. Note that the electrocardiographic signal processing section 512, the pulse wave signal processing section 523, and the arithmetic processing section 531 are configured by a microprocessor that performs arithmetic processing, a ROM that stores programs and data used in executing various kinds of processing by the microprocessor, a RAM that temporarily stores various data such as arithmetic operation results, a backup RAM in which data is backed up, and so on.

The electrocardiographic signal processing section 512 performs, for example, filtering processing to remove noise, normalizing processing, and the like on the electrocardiographic signal having undergone the A/D conversion. The electrocardiographic signal having been processed in the manner described above is outputted to the arithmetic processing section 531.

Meanwhile, the driving section 521 drives a light-emitting device, such as an LED, a VCSEL, or the like, that configures the photoelectric pulse wave sensor 21. The pulse wave signal amplifying section 522 is configured with an amplifier using an operational amplifier or the like, for example, and amplifies a photoelectric pulse wave signal detected by a light-receiving device, such as a photodiode, a phototransistor, or the like, that configures the photoelectric pulse wave sensor 21. The photoelectric pulse wave signal amplified by the pulse wave signal amplifying section 522 undergoes A/D conversion, thereafter is outputted to the pulse wave signal processing section 523.

The pulse wave signal processing section 523 performs, for example, filtering processing to remove noise, normalizing processing, and the like on the photoelectric pulse wave signal having undergone the A/D conversion. The photoelectric pulse wave signal having been processed in the manner described above is outputted to the arithmetic processing section 531.

The arithmetic processing section 531 detects peaks in the inputted electrocardiographic signal so as to obtain heart beats (R wave) and also obtain a heart rate from the intervals between the peaks. Further, the arithmetic processing section 531 calculates, for example, a heart-beat interval, a heart-beat interval fluctuation coefficient, and the like. In addition, the arithmetic processing section 531 detects peaks in the inputted photoelectric pulse wave signal so as to obtain pulse beats and also obtain a pulse rate from the intervals between the peaks. Moreover, the arithmetic processing section 531 calculates, for example, a pulse-beat interval, a pulse-beat interval variation coefficient, and the like.

In a case where an electrocardiographic signal cannot be obtained, for example, the arithmetic processing section 531 outputs a pulse rate based on the data of the photoelectric pulse wave signal in place of the electrocardiographic signal (in other words, complements the electrocardiographic signal). A peak of the electrocardiographic signal appears earlier than the corresponding peak of the photoelectric pulse wave signal. As such, by making use of this characteristic, the arithmetic processing section 531 may determine whether an obtained peak is a regular peak or noise. As described so far, by obtaining photoelectric pulse wave signals in addition to electrocardiographic signals, information of the heart rate and the like can be obtained more precisely and more stably.

The apparatus may be configured such that measurement data (measurement result) including a heart rate, a pulse rate, and so on having been obtained is outputted and displayed on a liquid crystal display (LCD) or the like. At this time, the apparatus may also be configured such that, for example, the above data is outputted and displayed on a mobile music player, a smartphone, or the like having a display. Further, the measurement data including a heart rate, a pulse rate, and so on having been obtained may be stored and accumulated in the aforementioned RAM or the like, for example, and may be outputted, after the measurement, to a personal computer or the like to obtain the above data.

Next, an electrocardiographic signal measurement method using the electrocardiographic signal measurement apparatus 1 will be described. In the case where an electrocardiographic signal and a photoelectric pulse wave signal are measured using the electrocardiographic signal measurement apparatus 1, one of the left and right outer ears is pinched with the outer ear clip 31 so that the outer ear electrode 11 and the photoelectric pulse wave sensor 21 are brought into contact with and attached to the above outer ear. At this time, the outer ear clip 31 (the outer ear electrode 11 and the photoelectric pulse wave sensor 21) is in contact with and attached to a portion near a boundary between the auricle and the earlobe of the outer ear.

Next, the upper arm belt 35 is wound around one of the left and right upper arms so that the upper arm electrode 15 is brought into contact with and attached to the stated upper arm. At this time, the upper arm belt 35 (the upper arm electrode 15) is attached to an upper arm on the opposite side in the horizontal direction to the side where the outer ear clip 31 (the outer ear electrode 11) is attached to the outer ear.

In the example in Fig. 2, the outer ear clip 31 (the outer ear electrode 11 and the photoelectric pulse wave sensor 21) is attached to the left ear, while the upper arm belt 35 (the upper arm electrode 15) is attached to the right upper arm. With the outer ear clip 31 (the outer ear electrode 11 and the photoelectric pulse wave sensor 21) and the upper arm belt 35 (the upper arm electrode 15) being attached in this manner, an electrocardiographic signal detected by the outer ear electrode 11 and the upper arm electrode 15 and a photoelectric pulse wave signal detected by the photoelectric pulse wave sensor 21 are measured. Since a measurement process of the electrocardiographic signal and so on is performed in the manner as described above, detailed description thereof is omitted herein.

According to the present embodiment, because the electrocardiographic electrodes (the outer ear electrode 11 and the upper arm electrode 15) are attached to an outer ear and an upper arm, clothes of a user need not be taken off when attaching/detaching the electrocardiographic electrodes 11 and 15, whereby the attaching/detaching will not take a long time. In addition, because the electrocardiographic electrodes 11 and 15 are attached to an outer ear and an upper arm, both hands are free, which makes it possible to carry out the measurement while making some other motions (for example, taking exercise or the like). Moreover, because the electrocardiographic electrodes 11 and 15 are attached to an outer ear and an upper arm, an electrocardiographic signal that is detected is relatively large and resistant to radiation noise from ambient environment, body motion noise, and so on, thereby making it possible to stably carry out the measurement. As a result, electrocardiographic signals can be measured in a stable manner all the time without obstructing motions of a user, and attaching/detaching the electrodes can be made with ease.

According to the present embodiment, the stability of measurement can be improved because the outer ear electrode 11 is attached to an outer ear, in particular, to a portion in the vicinity of a boundary between the earlobe and the auricle. In other words, variations in detected signals depending on individuals are smaller in comparison with a case in which the outer electrode 11 is attached to an earlobe of which shape considerably varies depending on individuals. Further, because the outer ear electrode 11 is attached to an area near a relatively hard auricle, the state of contact of the outer ear electrode 11 is stable even when taking exercise in comparison with a case in which the outer ear electrode 11 is attached to an earlobe which is softer. Accordingly, the stability of measurement of electrocardiographic signals can be improved.

According to the present embodiment, the upper arm electrode 15 is in contact with and attached to an upper arm on the opposite side in the horizontal direction to the side where the outer ear electrode 11 is attached to an outer ear, so that the amplitude of a detected electrocardiographic signal is larger, whereby the measurement of electrocardiographic signals can be more stably carried out. Further, the outer ear electrode 11 is made of a material having elasticity and conductivity and formed in a wave shape so as to bend along a contour of the outer ear so that the outer ear electrode 11 can be brought into close contact with the outer ear. With this, noise generated due to body motion or the like can be reduced and electrocardiographic signals can be more stably obtained. Furthermore, the upper arm electrode 15 is made of a material having elasticity and conductivity and is so formed as to be brought into close contact with and attached to an upper arm, whereby the upper arm electrode 15 can be in close contact with the upper arm. Accordingly, noise generated due to body motion or the like can be reduced and electrocardiographic signals can be more stably obtained.

According to the present embodiment, by combining and using the photoelectric pulse wave sensor 21, even if an electrocardiographic signal is hard to be measured due to, for example, an individual physical constitution, a state of contact between the skin and the electrocardiographic electrodes 11 and 15, and/or influence of disturbance noise, and so on, it is possible to complement a measurement result of electrocardiographic signal using a measurement result obtained by the photoelectric pulse wave sensor 21. Furthermore, in addition to information on a heart rate and heart-beat interval fluctuation, information on the degree of oxygen saturation, pulse wave transit time, and so on can be also obtained; moreover, the degree of fatigue, blood pressure fluctuation, and so on can be estimated based on these data.

### [Second Embodiment]

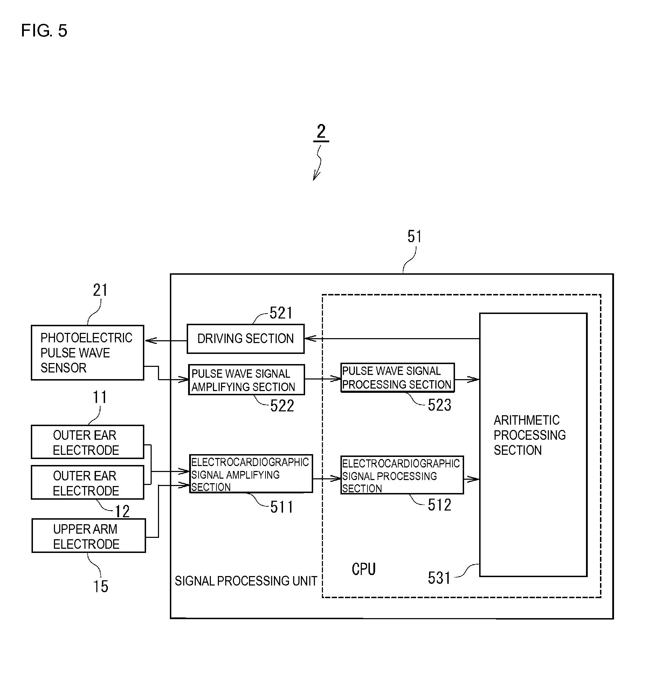

In the above embodiment, although the outer ear clip 31 (that is, the outer ear electrode 11) is attached to one of the left and right outer ears, the configuration may be configured such that two outer ear electrodes electrically connected with each other are attached to left and right outer ears, respectively. Here, a configuration of an electrocardiographic signal measurement apparatus 2 according to a second embodiment will be subsequently described with reference to Fig. 5. Fig. 5 is a block diagram illustrating the configuration of the electrocardiographic signal measurement apparatus 2. Note that in Fig. 5, constituent elements that are same as or equivalent to those in the first embodiment are given the same reference signs.

The electrocardiographic signal measurement apparatus 2 is different from the above-described electrocardiographic signal measurement apparatus 1 in a point that it has two outer electrodes, in place of the outer electrode 11, including an outer ear electrode 11 and an outer ear electrode 12 that are connected with each other (correspond to a first outer ear electrode and a second outer ear electrode disclosed in the appended claims). Since the other constituent elements are the same as or similar to those of the electrocardiographic signal apparatus 1, detailed description thereof will be omitted herein. The signal processing unit 51 processes potentials of the outer ears (electrocardiographic signals) inputted from the outer ear electrode 11 and the outer ear electrode 12 as well as a potential of an upper arm (electrocardiographic signal) inputted from the upper arm electrode 15 so as to measure a heart rate and so on. The configuration of the signal processing unit 51 is the same as the above-described one, detailed description thereof is omitted herein. It is sufficient that the photoelectric pulse wave sensor 21 and the signal processing unit 51 are only provided in one of the outer ear clips 31. However, the photoelectric pulse wave sensor 21 may be provided in the vicinity of the upper arm electrode 15 (for example, integrated as one entity).

In the case where a user measures an electrocardiographic signal using the electrocardiographic signal measurement apparatus 2, the left and right outer ears are first pinched with the two outer ear clips 31 so that the outer ear electrode 11 and the outer ear electrode 12 connected with each other are brought into contact with and attached to the left and right outer ears. At this time, the outer ear clips 31 (the outer ear electrode 11, the outer ear electrode 12) are each in contact with and attached to a portion near a boundary between the auricle and the earlobe of the outer ear.

Next, the upper arm belt 35 is wound around one of the left and right arms so that the upper arm electrode 15 is brought into contact with and attached to the upper arm.

With the outer ear clips 31 (the outer ear electrode 11, the outer ear electrode 12) and the upper arm belt 35 (the upper arm electrode 15) being attached in the manner described above, an electrocardiographic signal detected by the two outer ear electrodes 11, 12 connected with each other and the upper arm electrode 15 is measured. Since the measurement process of the electrocardiographic signal and so on is performed in the manner as described above, detailed description thereof is omitted herein.

According to the present embodiment, the two outer ear electrodes 11, 12 that are connected with each other to have the same potential are respectively brought into contact with and attached to the left and right outer ears, so that an electrocardiographic signal is detected between the two outer ear electrodes 11, 12 and the upper arm electrode 15. This makes it possible for electrocardiographic signals to be more stably measured.

### [Third Embodiment]

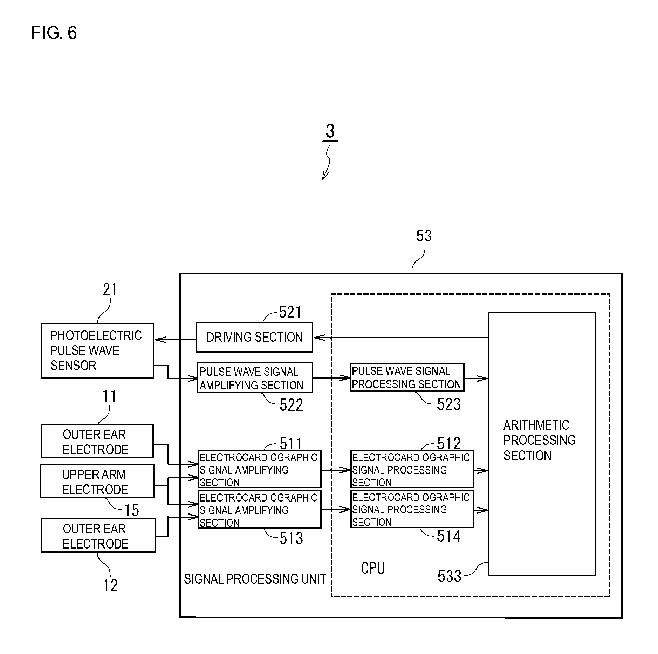

In the above-described second embodiment, although the electrocardiographic signal is measured using the two outer ear electrodes 11, 12 connected with each other and the upper arm electrode 15, the apparatus may have a configuration in which one outer ear electrode 11 and the upper arm electrode 15 are combined and the other outer ear electrode 12 and the upper arm electrode 15 are also combined so that an electrocardiographic signal is measured using the above combination sets of electrodes. As such, next, a configuration of an electrocardiographic signal measurement apparatus 3 according to a third embodiment will be described with reference to Fig. 6. Fig. 6 is a block diagram illustrating the configuration of the electrocardiographic signal measurement apparatus 3. Note that in Fig. 6, constituent elements that are same as or equivalent to those in the first embodiment are given the same reference signs.

As described above, the electrocardiographic signal measurement apparatus 3 has two outer ear electrodes, that is, the outer ear electrode 11 and the outer ear electrode 12, and the upper arm electrode 15. The outer ear electrode 11, the outer ear electrode 12, and the upper arm electrode 15 are connected with a signal processing unit 53. It is sufficient that the photoelectric pulse wave sensor 21 and the signal processing unit 53 are only provided in one of the outer ear clips 31. Note that, however, the photoelectric pulse wave sensor 21 may be provided in the vicinity of the upper arm electrode 15 (for example, integrated as one entity).

The signal processing unit 53 is different from the case of the electrocardiographic signal measurement apparatus 1 in a point that it includes, in addition to the electrocardiographic signal amplifying section 511 and the electrocardiographic signal processing section 512 for processing the electrocardiographic signal from the one outer ear electrode 11 (corresponds to a first outer ear electrode disclosed in the appended claim) and the upper arm electrode 15, an electrocardiographic signal amplifying section 513 and an electrocardiographic signal processing section 514 for processing the electrocardiographic signal from the other outer ear electrode 12 (corresponds to a second outer ear electrode disclosed in the appended claims) and the upper arm electrode 15. Since the electrocardiographic signal amplifying section 513 and the electrocardiographic signal processing section 514 are the same as the aforementioned electrocardiographic signal amplifying section 511 and electrocardiographic signal processing section 512, respectively, detailed description thereof is omitted herein.

The signal processing unit 53 is different from the case of the electrocardiographic signal measurement apparatus 1 in a point that it includes an arithmetic processing section 533 configured to select, of the combination of the outer ear electrode 11 and upper arm electrode 15 and the combination of the outer ear electrode 12 and upper arm electrode 15, the combination which detects an electrocardiographic signal with a larger SN ratio and then measure the electrocardiographic signal. The other constituent elements are the same as or similar to those in the case of the electrocardiographic signal measurement apparatus 1, therefor detailed description thereof is omitted herein. The arithmetic processing section 533 may perform the combination selection through comparing detection rates of electrocardiographic peaks, peak amplitude, noise levels, and the like, for example, in place of or in addition to comparing SN ratios.

In the case where a user measures an electrocardiographic signal using the electrocardiographic signal measurement apparatus 3, the left and right outer ears are first pinched with the two outer ear clips 31 so that the outer ear electrode 11 and the outer ear electrode 12 are brought into contact with and attached to the left and right outer ears. At this time, the outer ear clips 31 (the outer ear electrode 11, the outer ear electrode 12) are each attached to a portion in the vicinity of a boundary between the auricle and the earlobe of the outer ear.

Subsequently, the upper arm belt 35 is wound around one of the left and right upper arms so that the upper arm electrode 15 is brought into contact with and attached to the upper arm.

With the outer ear clips 31 (the outer ear electrode 11, the outer ear electrode 12) and the upper arm belt 35 (the upper arm electrode 15) being attached in the manner described above, of the combination of the outer ear electrode 11 and upper arm electrode 15 and the combination of the outer ear electrode 12 and upper arm electrode 15, the combination that detects an electrocardiographic signal with a larger SN ratio is selected, and the electrocardiographic signal is then measured.

According to the present embodiment, a SN ratio of the electrocardiographic signal between the right outer ear and an upper arm and a SN ration of the electrocardiographic signal between the left outer ear and the upper arm are compared with each other, and the combination that exhibits a larger SN ratio is selected so as to measure the electrocardiographic signal. This makes it possible for electrocardiographic signals to be more stably measured.

### [Fourth Embodiment]

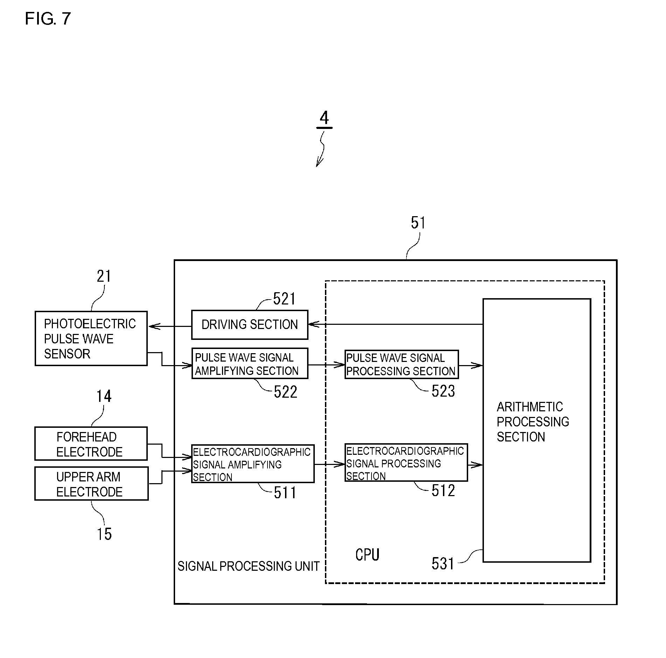

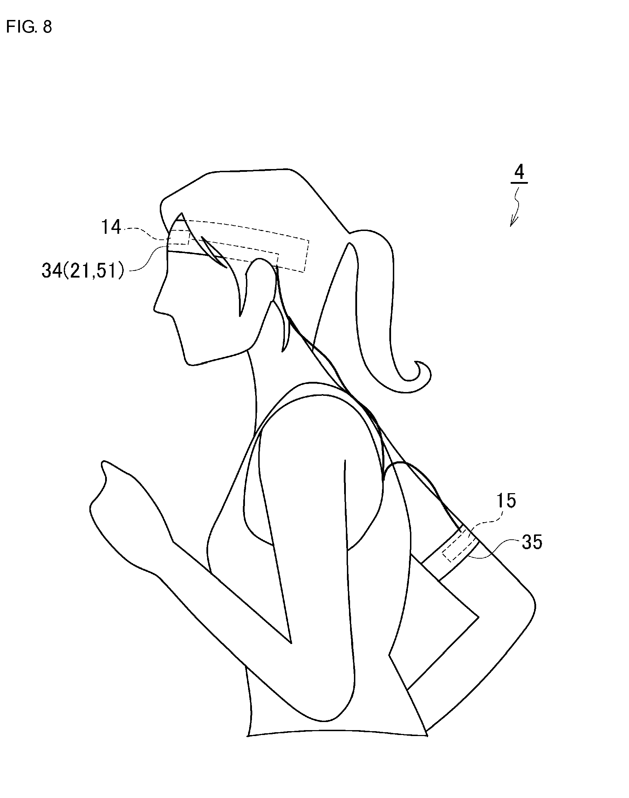

In the aforementioned first embodiment, although the electrocardiographic signal is measured using the outer ear electrode 11 attached to an outer ear and the upper arm electrode 15, the apparatus may be configured such that an electrocardiographic signal is measured using a forehead electrode 14 attached to a forehead and the upper arm electrode 15. Such that, next, a configuration of an electrocardiographic signal measurement apparatus 4 according to a fourth embodiment will be described referring to Figs. 7 and 8. Fig. 7 is a block diagram illustrating the configuration of the electrocardiographic signal measurement apparatus 4. Meanwhile, Fig. 8 is a diagram illustrating a mounting example of the electrocardiographic signal measurement apparatus 4. In Figs. 7 and 8, constituent elements that are same as or equivalent to those in the first embodiment are given the same reference signs.

The electrocardiographic signal measurement apparatus 4 is different from the electrocardiographic signal measurement apparatus 1 in a point that it has the forehead electrode 14 to be attached to a forehead in place of the outer electrode 11. The other constituent elements are the same as or similar to those of the electrocardiographic signal measurement apparatus 1, therefore detailed description thereof is omitted herein. Note that the photoelectric pulse wave sensor 21 is provided in the vicinity of the forehead electrode 14 or the upper arm electrode 15 (for example, integrated as one entity).

The forehead electrode 14 is made of, for example, conductive rubber, conductive cloth, or the like, and is provided on the inner side of a headband 34. Accordingly, as shown in Fig. 8, by attaching the headband 34 to the forehead of a user, the forehead electrode 14 is attached to the forehead of the user so as to be in contact with the forehead thereof. The forehead electrode 14 is connected with the signal processing unit 51 so as to output a potential of the forehead (electrocardiographic signal) to the signal processing unit 51.

The signal processing unit 51 processes a potential of the forehead (electrocardiographic signal) inputted from the forehead electrode 14 and a potential of an upper arm (electrocardiographic signal) inputted from the upper arm electrode 15 so as to measure a heart rate and so on. The configuration of the signal processing unit 51 is the same as the above-described one, therefore detailed description thereof is omitted herein.

In the case where a user measures an electrocardiographic signal using the electrocardiographic signal measurement apparatus 4, the headband 34 is first attached to the head, as shown in Fig. 8, so that the forehead electrode 14 is brought into contact with and attached to the forehead.

Subsequently, the upper arm belt 35 is wound around one of the left and right upper arms so that the upper arm electrode 15 is brought into contact with and attached to the upper arm.

With the headband 34 (the forehead electrode 14) and the upper arm belt 35 (the upper arm electrode 15) being attached in the manner described above, an electrocardiographic signal detected by the forehead electrode 14 and the upper arm electrode 15 is measured. Since the measurement process of the electrocardiographic signal and so on is performed in the manner as described above, detailed description thereof is omitted herein.

According to the present embodiment, because the electrocardiographic electrodes (the forehead electrode 14 and the upper arm electrode 15) are attached to the forehead and an upper arm, clothes of a user need not be taken off when attaching/detaching the electrocardiographic electrodes 14 and 15, whereby the attaching/detaching will not take a long time. In addition, because the electrocardiographic electrodes 14 and 15 are attached to the forehead and an upper arm, both hands are free, which makes it possible to carry out the measurement while making some other motions (for example, taking exercise or the like). Moreover, because the electrocardiographic electrodes 14 and 15 are attached to the forehead and an upper arm, an electrocardiographic signal that is detected is relatively large and resistant to radiation noise from ambient environment, body motion noise, and so on, thereby making it possible to stably carry out the measurement. As a result, electrocardiographic signals can be measured in a stable manner all the time without obstructing motions of the user, and attaching/detaching the electrodes can be made with ease.

### [Fifth Embodiment]

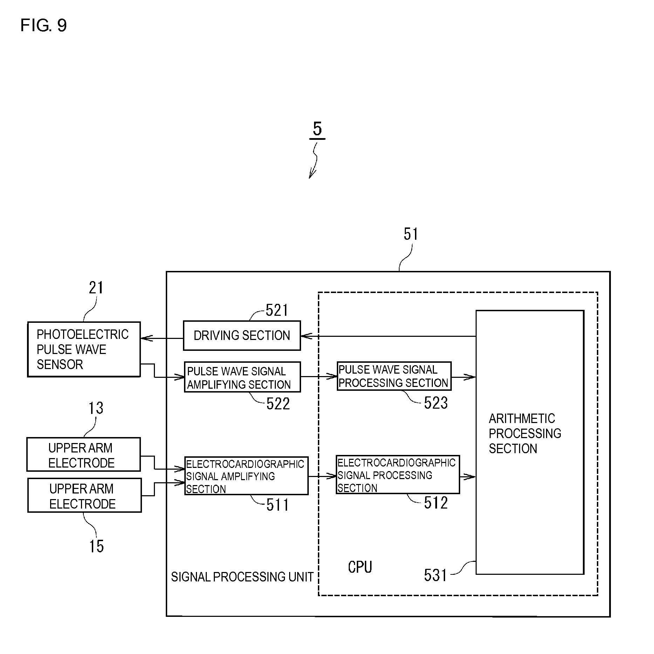

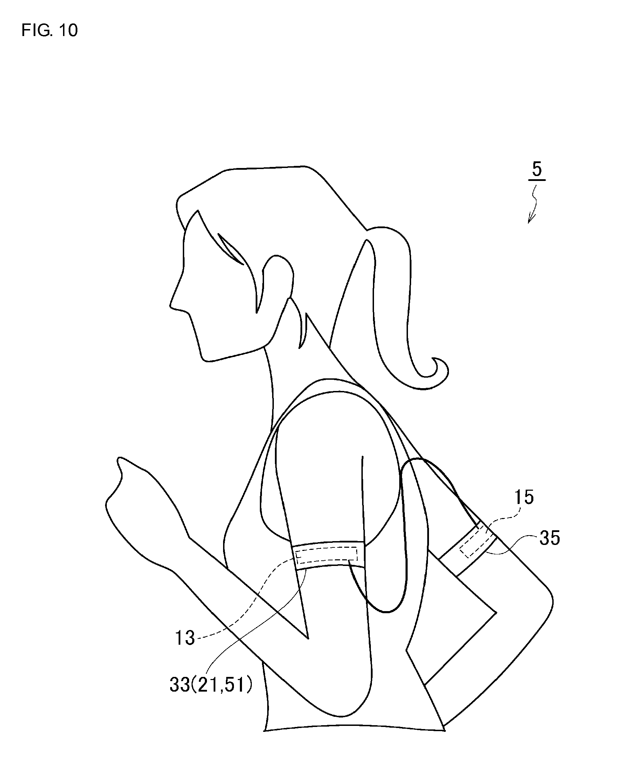

In the first embodiment, although the electrocardiographic signal is measured using the outer ear electrode 11 attached to an outer ear and the upper arm electrode 15, the apparatus may also be configured such that an electrocardiographic signal is measured using two upper arm electrodes, that is, an upper arm electrode 13 and the upper arm electrode 15 being attached to the left and right upper arms. As such, next, a configuration of an electrocardiographic signal measurement apparatus 5 according to a fifth embodiment will be described referring to Figs. 9 and 10. Fig. 9 is a block diagram illustrating the configuration of the electrocardiographic signal measurement apparatus 5. Meanwhile, Fig. 10 is a diagram illustrating a mounting example of the electrocardiographic signal measurement apparatus 5. In Figs. 9 and 10, constituent elements that are same as or equivalent to those in the first embodiment are given the same reference signs.

The electrocardiographic signal measurement apparatus 5 is different from the electrocardiographic signal measurement apparatus 1 in a point that it has the second upper arm electrode 13 to be attached to an upper arm in place of the outer electrode 11. The other constituent elements are the same as or similar to those of the electrocardiographic signal apparatus 1, therefore detailed description thereof is omitted herein. Note that the photoelectric pulse wave sensor 21 is provided in the vicinity of the upper arm electrode 13 or the upper arm electrode 15 (for example, integrated as one entity).

The upper arm electrode 13 and the upper arm electrode 15 are configured in the same manner. In other words, the upper arm electrode 13 is made of, for example, conductive rubber, conductive cloth, or the like, and is provided on the inner side of an upper arm belt 33. Accordingly, by winding and attaching the upper arm belt 33 around an upper arm of a user using a hook-and-loop fastener or the like, for example, the upper arm electrode 13 is attached to the upper arm of the user so as to be in contact with the upper arm thereof. The upper arm electrode 13 is connected with the signal processing unit 51 so as to output a potential (electrocardiographic signal) of an upper arm (left upper arm in the example in Fig. 10) to the signal processing unit 51.

The signal processing unit 51 processes potentials of the left and right upper arms (electrocardiographic signals) inputted from the two upper arm electrodes 13 and 15 so as to measure a heart rate and so on. The configuration of the signal processing unit 51 is the same as the above-described one, therefore detailed description thereof is omitted herein.

In the case where a user measures an electrocardiographic signal using the electrocardiographic signal measurement apparatus 5, the upper arm belt 33 is first wound around, for example, the left upper arm as shown in Fig. 10, so that the upper arm electrode 13 is brought into contact with and attached to the left upper arm.

Subsequently, the upper arm belt 35 is wound around the right upper arm, so that the upper arm electrode 15 is brought into contact with and attached to the right upper arm.

With the upper arm belt 33 (the upper arm electrode 13) and the upper arm belt 35 (the upper arm electrode 15) being attached in the manner described above, an electrocardiographic signal detected by the upper arm electrode 13 and the upper arm electrode 15 is measured. Since the measurement process of the electrocardiographic signal and so on is performed in the manner as described above, detailed description thereof is omitted herein.

According to the present embodiment, because the electrocardiographic electrodes (the upper arm electrode 13 and the upper arm electrode 15) are attached to left and right upper arms, clothes of a user need not be taken off when attaching/detaching the electrocardiographic electrodes 13 and 15, whereby the attaching/detaching will not take a long time. In addition, because the electrocardiographic electrodes 13 and 15 are attached to the left and right upper arms, both hands are free, which makes it possible to carry out the measurement while making some other motions (for example, taking exercise or the like). Moreover, because the electrocardiographic electrodes 13 and 15 are attached to the left and right upper arms, an electrocardiographic signal that is detected is relatively large and resistant to radiation noise from ambient environment, body motion noise, and so on, thereby making it possible to stably carry out the measurement. As a result, electrocardiographic signals can be measured in a stable manner all the time without obstructing motions of a user, and attaching/detaching the electrodes can be made with ease.

Thus far, the embodiments of the present invention have been described. However, the present invention is not intended to be limited to the above-described embodiments, and various kinds of variations can be made thereupon. For example, in the above embodiments, although the upper arm electrodes 13 and 15 are attached to the upper arms of a user, these electrodes may be attached to the shoulders thereof.

In the above embodiments, although the photoelectric pulse wave sensor 21 is provided in addition to the outer ear electrode 11, the photoelectric pulse wave sensor 21 is not necessarily needed and can be omitted. Further, in the above embodiments, although the photoelectric pulse wave sensor 21 and the signal processing unit 51 are integrally formed as one entity, they may be isolated from each other.

In the fourth embodiment, although the forehead electrode 14 is provided on the inner side of the headband 34, the forehead electrode 14 may be provided, for example, on the inner side of a hat instead of the headband 34. Thus, when a user puts on this hat, the forehead electrode 14 is brought into contact with and attached to the forehead of the user.

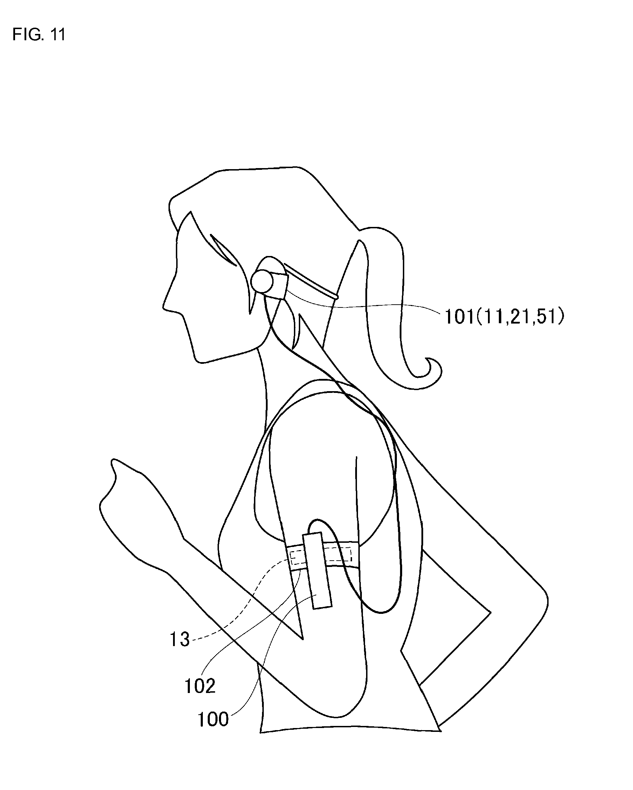

As described earlier, the apparatus may be configured such that the measurement data of a heart rate, a pulse rate, and so on having been obtained is outputted and displayed on a mobile music player 100, a smartphone, or the like equipped with a display, as shown in Fig. 11. In this case, the electrocardiographic signal processing section 512, the pulse wave signal processing section 523, and the arithmetic processing section 531, for example, may be assembled inside the mobile music player 100 or the smartphone. Further, at this time, the apparatus may be configured such that the outer ear electrode 11 (12) is provided in a headset 101 or an earphone connected with the mobile music player 100 or the like in place of the outer ear clip 31, so that the outer ear electrode 11 (12) makes contact with an outer ear when a user puts on the headset 101 or the earphone. Furthermore, the upper arm electrode 13 may be provided on the inner surface of a belt 102 arranged to prepare a pocked, on an upper arm, for holding the mobile music player 100 or the like.

### Reference Signs List

1, 2, 3, 4, 5 electrocardiographic signal measurement apparatus
11, 12 outer ear electrode
13, 15 upper arm electrode
14 forehead electrode
21 photoelectric pulse wave sensor
31 outer ear clip
33, 35 upper arm belt
34 headband
51, 53 signal processing unit
531, 533 arithmetic processing section

## Claims

1. An electrocardiographic signal measurement apparatus comprising:
an outer ear electrode that is in contact with and attached to an outer ear;
an upper arm / shoulder electrode that is in contact with and attached to an upper arm or a shoulder; and
a measurement means that is connected with the outer ear electrode and the upper arm / shoulder electrode and measures an electrocardiographic signal detected by the outer ear electrode and the upper arm / shoulder electrode.

2. The electrocardiographic signal measurement apparatus according to Claim 1,
wherein the outer ear electrode is in contact with and attached to an area including a boundary region between an auricle and an earlobe configuring the outer ear.

3. The electrocardiographic signal measurement apparatus according to Claim 1 or 2,
wherein the outer ear electrode is made of a material having elasticity and conductivity and formed so as to bend along a contour of the outer ear.

4. The electrocardiographic signal measurement apparatus according to any one of Claims 1 through 3,
wherein the upper arm / shoulder electrode is in contact with and attached to an upper arm or a shoulder on the opposite side in the horizontal direction to a side where the outer ear electrode is attached to the outer ear.

5. The electrocardiographic signal measurement apparatus according to any one of Claims 1 through 4,
wherein the upper arm / shoulder electrode is made of a material having elasticity and conductivity and formed so as to be in close contact with and attached to an upper arm or a shoulder.

6. The electrocardiographic signal measurement apparatus according to any one of Claims 1 through 5, further comprising:
a photoelectric pulse wave sensor that is provided in the vicinity of the outer ear electrode or the upper arm / shoulder electrode and detects a photoelectric pulse wave signal,
wherein the measurement means measures an electrocardiographic signal and complements the electrocardiographic signal using the photoelectric pulse wave signal detected by the photoelectric pulse wave sensor.

7. An electrocardiographic signal measurement apparatus comprising:
a first outer ear electrode and a second outer ear electrode that are electrically connected with each other and are in contact with and attached to left and right outer ears;
an upper arm / shoulder electrode that is in contact with and attached to an upper arm or a shoulder; and
a measurement means that is connected with the first outer ear electrode, the second outer ear electrode, and the upper arm / shoulder electrode so as to measure an electrocardiographic signal detected by the first outer ear electrode, the second outer ear electrode, and the upper arm / shoulder electrode.

8. An electrocardiographic signal measurement apparatus comprising:
a first outer ear electrode that is in contact with and attached to one of the left and right outer ears;
a second outer ear electrode that is in contact with and attached to the other outer ear;
an upper arm / shoulder electrode that is in contact with and attached to an upper arm or a shoulder; and
a measurement means that is connected with the first outer ear electrode, the second outer ear electrode, and the upper arm / shoulder electrode and performs electrocardiographic signal measurement in a manner in which, of a combination of the first outer ear electrode and the upper arm / shoulder electrode and a combination of the second outer ear electrode and the upper arm / shoulder electrode, the combination that detects an electrocardiographic signal with a larger SN ratio is selected and the electrocardiographic signal is then measured.

9. The electrocardiographic signal measurement apparatus according to Claim 7 or 8, further comprising:
a photoelectric pulse wave sensor that is provided in the vicinity of the first outer ear electrode, the second outer ear electrode, or the upper arm / shoulder electrode and detects a photoelectric pulse wave signal,
wherein the measurement means measures an electrocardiographic signal and complements the electrocardiographic signal using a photoelectric pulse wave signal detected by the photoelectric pulse wave sensor.

10. An electrocardiographic signal measurement apparatus comprising:
a forehead electrode that is in contact with and attached to a forehead;
an upper arm / shoulder electrode that is in contact with and attached to an upper arm or a shoulder; and
a measurement means configured to measure an electrocardiographic signal detected by the forehead electrode and the upper arm / shoulder electrode.

11. The electrocardiographic signal measurement apparatus according to Claim 10, further comprising:
a photoelectric pulse wave sensor that is provided in the vicinity of the forehead electrode or the upper arm / shoulder electrode and detects a photoelectric pulse wave signal,
wherein the measurement means measures an electrocardiographic signal and complements the electrocardiographic signal using a photoelectric pulse wave signal detected by the photoelectric pulse wave sensor.

12. An electrocardiographic signal measurement apparatus comprising:
a first upper arm / shoulder electrode that is in contact with and attached to one of the left and right upper arms or shoulders;
a second upper arm / shoulder electrode that is in contact with and attached to the other upper arm or shoulder; and
a measurement means configured to measure an electrocardiographic signal detected by the first upper arm / shoulder electrode and the second upper arm / shoulder electrode.

13. The electrocardiographic signal measurement apparatus according to Claim 12, further comprising:
a photoelectric pulse wave sensor that is provided in the vicinity of the first upper arm / shoulder electrode or the second upper arm / shoulder electrode and detects a photoelectric pulse wave signal,
wherein the measurement means measures an electrocardiographic signal and complements the electrocardiographic signal using a photoelectric pulse wave signal detected by the photoelectric pulse wave sensor.

14. An electrocardiographic signal measurement method comprising:
an outer ear electrode attaching step in which an outer ear electrode is brought into contact with and attached to an outer ear;
an upper arm / shoulder electrode attaching step in which an upper arm / shoulder electrode is brought into contact with and attached to an upper arm or a shoulder; and
a measuring step in which an electrocardiographic signal detected by the outer ear electrode and the upper arm / shoulder electrode is measured.

15. The electrocardiographic signal measurement method according to Claim 14,
wherein in the outer ear electrode attaching step, the outer ear electrode is brought into contact with and attached to an area including a boundary region between an auricle and an earlobe configuring the outer ear.

16. The electrocardiographic signal measurement method according to Claim 14 or 15,
wherein in the upper arm / shoulder electrode attaching step, the upper arm / shoulder electrode is brought into contact with and attached to an upper arm or a shoulder on the opposite side in the horizontal direction to the side where the outer ear electrode is attached to the outer ear.

17. An electrocardiographic signal measurement method comprising:
an outer ear electrode attaching step in which a first outer ear electrode and a second outer ear electrode electrically connected with each other are brought into contact with and attached to left and right outer ears;
an upper arm / shoulder electrode attaching step in which an upper arm / shoulder electrode is brought into contact with and attached to an upper arm or a shoulder; and
a measuring step in which measured is an electrocardiographic signal detected by the first outer ear electrode, the second outer ear electrode, and the upper arm / shoulder electrode.

18. The electrocardiographic signal measurement method according to any one of Claims 14 through 17,
wherein in the outer ear electrode attaching step, a photoelectric pulse wave sensor for detecting a photoelectric pulse wave signal is further brought into contact with and attached to the outer ear; and
in the measuring step, an electrocardiographic signal is measured and complemented using a photoelectric pulse wave signal detected by the photoelectric pulse wave sensor.

19. An electrocardiographic signal measurement method comprising:
a first outer ear electrode attaching step in which a first outer ear electrode is brought into contact with and attached to one of the left and right outer ears;
a second outer ear electrode attaching step in which a second outer ear electrode is brought into contact with and attached to the other outer ear;
an upper arm / shoulder electrode attaching step in which an upper arm / shoulder electrode is brought into contact with and attached to an upper arm or a shoulder; and
a measuring step in which electrocardiographic signal measurement is performed in a manner in which, of a combination of the first outer ear electrode and the upper arm / shoulder electrode and a combination of the second outer ear electrode and the upper arm / shoulder electrode, the combination that detects an electrocardiographic signal with a larger SN ratio is selected and the electrocardiographic signal is then measured.

20. An electrocardiographic signal measurement method comprising:
a forehead electrode attaching step in which a forehead electrode is brought into contact with and attached to a forehead;
an upper arm / shoulder electrode attaching step in which an upper arm / shoulder electrode is brought into contact with and attached to an upper arm or a shoulder; and
a measuring step in which an electrocardiographic signal detected by the forehead electrode and the upper arm / shoulder electrode is measured.

21. An electrocardiographic signal measurement method comprising:
a first upper arm / shoulder electrode attaching step in which a first upper arm / shoulder electrode is brought into contact with and attached to one of the left and right upper arms or shoulders;
a second upper arm / shoulder electrode attaching step in which a second upper arm / shoulder electrode is brought into contact with and attached to the other upper arm or shoulder; and
a measuring step in which an electrocardiographic signal detected by the first upper arm / shoulder electrode and the second upper arm / shoulder electrode is measured.
